# EUROPEAN PATENT SPECIFICATION

(11) **EP 4 647 110 B1**
(45) Date of publication and mention of the grant of the patent: **14.10.2026**
(21) Application number: 25174891.9
(22) Date of filing: 07.05.2025
(51) Int. Cl.: A61N 1/05, A61N 1/375

(54) **BIOSTIMULATOR HEADER ASSEMBLY HAVING INTEGRATED FIXATION ELEMENT MOUNT**
BIOSTIMULATOR-HEADER-ANORDNUNG MIT INTEGRIERTER BEFESTIGUNGSELEMENTHALTERUNG
ENSEMBLE COLLECTEUR DE BIOSTIMULATEUR AYANT UN SUPPORT D'ÉLÉMENT DE FIXATION INTÉGRÉ

(30) Priority: 08.05.2024 US 202463644439 P; 06.05.2025 US 202519200552
(43) Date of publication of application: 12.11.2025
(73) Proprietor: Pacesetter, Inc., Sylmar, CA 91342 (US)
(72) Inventor: Park, Jason, Sylmar, CA 91342 (US); Garabed, Arees, Sylmar, CA 91342 (US); Sahabi, Kavous, Sylmar, CA 91342 (US); Xie, Jeffrey, Sylmar, CA 91342 (US); Soriano, Alex, Sylmar, CA 91342 (US)
(74) Representative: Barker Brettell Sweden AB

(56) References cited:
- US-A1- 2021 220 657
- US-A1- 2021 228 888
- US-A1- 2023 285 757

## Description

### TECHNICAL FIELD

The present disclosure relates to biostimulators having header assemblies. More specifically, the present disclosure relates to leadless biostimulators having header assemblies that include a mount for a fixation element.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Cardiac pacing by currently available or conventional pacemakers is usually performed by a pulse generator implanted subcutaneously or sub-muscularly in or near a patient's pectoral region. The pulse generator usually connects to the proximal end of one or more implanted leads through a feedthrough assembly, which creates an isolated electrical pass-through into a hermetic case for pulse/sense transmissions to a target tissue. The feedthrough assembly can be used in low voltage or high voltage applications. A distal end of the implanted leads, which typically have lengths of 50 to 70 centimeters, contains one or more electrodes for positioning adjacent to the inside or outside wall of a cardiac chamber. The leads have an insulated electrical conductor or conductors for connecting the pulse generator to the electrodes in the heart. Accordingly, the pulse generator can deliver a pacing pulse from within a hermetically sealed housing through the feedthrough assembly, the lead, and the electrode to the target tissue.

Conventional pacemakers have several drawbacks, including a risk of lead or feedthrough assembly breakage, complex connections between the leads and the feedthrough assembly, and a risk of infection and morbidity due to the separate leads and pulse generator components. Many of the issues associated with conventional pacemakers are resolved by the development of a self-contained and self-sustainable biostimulator, or so-called leadless biostimulator. The leadless biostimulator can be attached to tissue within a dynamic environment, e.g., within a chamber of a beating heart, to deliver pacing pulses directly to the tissue without the use of leads.

Leadless biostimulators include feedthrough components to provide electrical connection between conducting components, such as pacing electrodes, and internal circuitry. The feedthrough components maintain electrical isolation to other components, and require hermeticity to avoid fluid and electrical leaks.

US2023/285757 A1 discloses a leadless biostimulator having a threaded connection to a flange.

### SUMMARY

Existing leadless biostimulators can have a header assembly that supports the device at a target site using a fixation element. Interconnections between header assembly components are intended to hermetically seal internal circuitry from a surrounding environment. For example, the fixation element can be connected to a mount by a threaded connection, e.g., screwed onto a holding thread of the mount, and the mount in turn may be screwed onto a thread of a flange. The holding thread of the mount and the thread of the feedthrough assembly can be machined, and requires a minimum length to obtain a secure and hermetic attachment, thereby increasing device size. Hermeticity and electrical isolation can also be achieved using gaskets and adhesive that also increase device size and complexity. More particularly, the gaskets and adhesives are additional components, having respective costs and assembly complexities, as well as potential electrical or mechanical failure pathways. Accordingly, existing leadless biostimulators can benefit from a header assembly that includes threadless attachments and fewer manufacturing processes or components to reduce device size and cost, and to increase mechanical stability and electrical reliability, leading to improved device implantation and manufacturability characteristics.

A header assembly for a biostimulator, e.g., a leadless biostimulator, having an integrated fixation element mount is described. In an embodiment, the header assembly includes a flange having a central axis. The header assembly includes a fixation element mount mounted on the flange. The fixation element mount includes a mounting ring on an insulator base. The mounting ring extends around the central axis within a groove of the insulator base. The header assembly includes a fixation element coupled to the mounting ring.

A biostimulator is described. In an embodiment, the biostimulator includes a housing having a central axis and an electronics compartment. The biostimulator includes an electronics assembly mounted in the electronics compartment. The biostimulator includes a header assembly including a flange mounted on the housing along the central axis. The header assembly includes a fixation element mount mounted on the flange. The fixation element mount includes a mounting ring on an insulator base. The mounting ring extends around the central axis within a groove of the insulator base. The header assembly includes a fixation element coupled to the mounting ring.

A biostimulator system is described. In an embodiment, the biostimulator system includes a biostimulator transport system having a distal end. The biostimulator system includes a biostimulator coupled to the distal end of the biostimulator transport system. The biostimulator includes a flange having a central axis, and a fixation element mount mounted on the flange. The fixation element mount includes a mounting ring on an insulator base. The mounting ring extends around the central axis within a groove of the insulator base. The biostimulator includes a fixation element coupled to the mounting ring.

### BRIEF DESCRIPTION OF THE DRAWINGS

The novel features of implementations of the present disclosure are set forth with particularity in the claims that follow. A better understanding of the features and advantages of such implementations will be obtained by reference to the following detailed description that sets forth illustrative examples in which the principles of the disclosure are utilized, and the accompanying drawings of which:
FIG. 1 is a perspective view of a biostimulator, in accordance with an embodiment.
FIG. 2 is a perspective view of a biostimulator system, in accordance with an embodiment.
FIG. 3 is a perspective view of a header assembly, in accordance with an embodiment.
FIG. 4 is an exploded view of a header assembly, in accordance with an embodiment.
FIG. 5 is a cross-sectional view of a header assembly, in accordance with an embodiment.
FIG. 6 is an exploded view of a fixation element mount, in accordance with an embodiment.
FIG. 7 is an endview of a header assembly, in accordance with an embodiment.
FIG. 8 is a perspective view of a header assembly, in accordance with an embodiment.
FIG. 9 is an exploded view of a header assembly, in accordance with an embodiment.
FIG. 10 is a cross-sectional view of a header assembly, in accordance with an embodiment.
FIG. 11 is a perspective view of a fixation element mount, in accordance with an embodiment.
FIG. 12 is a cross-sectional view of an insulator base, in accordance with an embodiment.
FIG. 13 is a perspective view of a mounting ring, in accordance with an embodiment.
FIG. 14 is a top view of a mounting ring, in accordance with an embodiment.

### DETAILED DESCRIPTION

Implementations of the present disclosure include a biostimulator, e.g., a leadless cardiac pacemaker, having a header assembly that includes an integrated fixation element mount. The biostimulator may be used to pace cardiac tissue. The biostimulator may be used in other applications, however, such as deep brain stimulation. Thus, reference to the biostimulator as being a cardiac pacemaker is not limiting.

Descriptions of various implementations of the present disclosure are made with reference to the figures. However, certain implementations may be practiced without one or more of these specific details, or in combination with other known methods and configurations. In the following description, numerous specific details are set forth, such as specific configurations, dimensions, and processes, in order to provide a thorough understanding of the example implementations. In other instances, well-known processes and manufacturing techniques have not been described in particular detail in order to not unnecessarily obscure the description. Reference throughout this specification to "one implementation," "an implementation," or the like, means that a particular feature, structure, configuration, or characteristic described is included in at least one implementation. Thus, the appearance of the phrase "one implementation," "an implementation," or the like, in various places throughout this specification are not necessarily referring to the same implementation. Furthermore, the particular features, structures, configurations, or characteristics may be combined in any suitable manner in one or more implementations, as long as they fall within the scope of the appended claims.

The use of relative terms throughout the description may denote a relative position or direction. For example, "distal" may indicate a first direction along a central axis of a biostimulator. Similarly, "proximal" may indicate a second direction opposite to the first direction. Such terms are provided to establish relative frames of reference, however, and are not intended to limit the use or orientation of a biostimulator or a biostimulator system to a specific configuration described in the various implementations below.

In an aspect of the present disclosure, a biostimulator including an integrated fixation element mount is provided. The integrated fixation element mount includes a mounting ring integrated with an insulator base. For example, the mounting ring can be located on the insulator base, e.g., in a groove of the insulator base. A fixation element may be directly connected, e.g., welded, to the mounting ring. Furthermore, the insulator base can be directly connected, e.g., by a brazing joint, to a flange. The direct connections by thermal bonds can eliminate threaded connections and seal components that may otherwise be needed to secure the components and hermetically seal the biostimulator. Accordingly, the integrated fixation element mount and direct connections can reduce overall header assembly length and miniaturize the biostimulator while reducing the risk of fluid and/or electrical leaks.

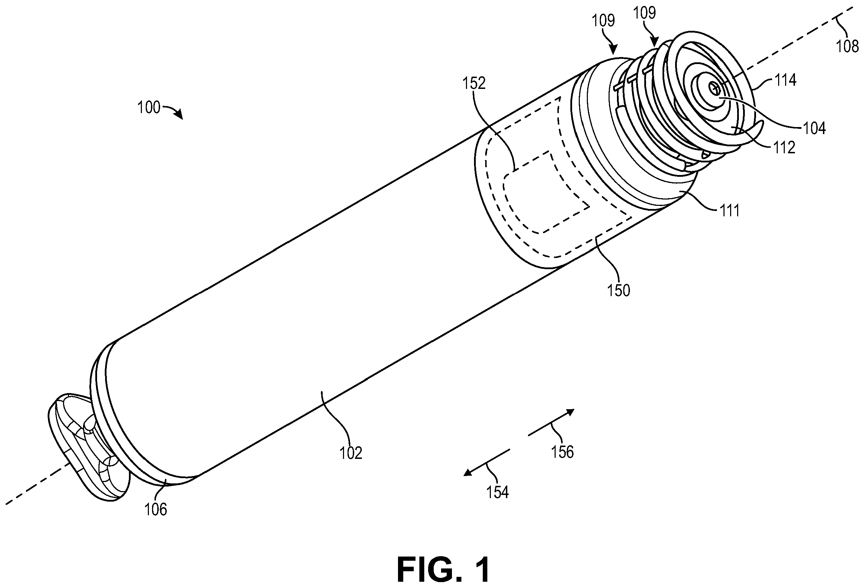

Referring to FIG. 1, a perspective view of a biostimulator is shown in accordance with an embodiment. The biostimulator 100 can be a leadless biostimulator, e.g., a leadless cardiac pacemaker. The biostimulator 100 can include a housing 102 having pacing electrodes. For example, the biostimulator 100 includes each of a distal electrode 104 and a proximal electrode 106 disposed on or integrated into the housing 102. The electrodes 104, 106 can be integral to the housing 102 or connected to the housing 102, e.g., at a distance of less than several centimeters from the housing 102. The housing 102 can contain an energy source (not shown) to provide power to the pacing electrodes 104, 106. The energy source can be, for example, a battery, such as a lithium carbon monofluoride (CFx) cell, or a hybrid battery, such as a combined CFx and silver vanadium oxide (SVO/CFx) mixed-chemistry cell. Similarly, the energy source can be an ultracapacitor. In one implementation, the energy source can be an energy harvesting device, such as a piezoelectric device that converts mechanical strain into electrical current or voltage. The energy source can also be an ultrasound transmitter that uses ultrasound technology to transfer energy from an ultrasound subcutaneous pulse generator to a receiver-electrode implanted on an endocardial wall.

A header assembly 109 can be mounted on the housing 102. The header assembly 109 can include a flange 111, and the flange 111 can have a longitudinal, central axis 108. The central axis 108 may be an axis of symmetry along which several other biostimulator components are disposed. For example, the header assembly 109 can be mounted on a distal end of the housing 102 along the central axis 108, and a fixation element 114 can be located along the central axis 108. The header assembly 109 can include a fixation element mount 112 mounted on the flange 111, and the fixation element 114 mounted on the fixation element mount 112. An electrical feedthrough in the header assembly 109 can include the distal electrode 104 insulated within the fixation element mount 112. The assembled components of the header assembly 109 can provide a distal region of the biostimulator 100 that attaches to a target tissue, e.g., via engagement of the fixation element 114 with the target tissue. The distal region can deliver a pacing impulse to the target tissue, e.g., via the distal electrode 104 that is held against the target tissue.

The housing 102 can have an electronics compartment 150 (shown by hidden lines). More particularly, the electronics compartment 150 can be a cavity laterally surrounded by a housing wall, e.g., a cylindrical wall, extending around the central axis 108. The housing wall can include a conductive, biocompatible, inert, and anodically safe material such as titanium, 316L stainless steel, or other similar materials, to laterally enclose the electronics compartment 150 between the energy source of the biostimulator 100 within a proximal portion of the housing 102, and the header assembly 109 at the distal portion of the biostimulator 100. More particularly, an energy source container can proximally enclose the electronics compartment 150 and the header assembly 109 can distally enclose the electronics compartment 150. The header assembly 109, the housing wall, and the power source container can surround a volume of the electronics compartment 150.

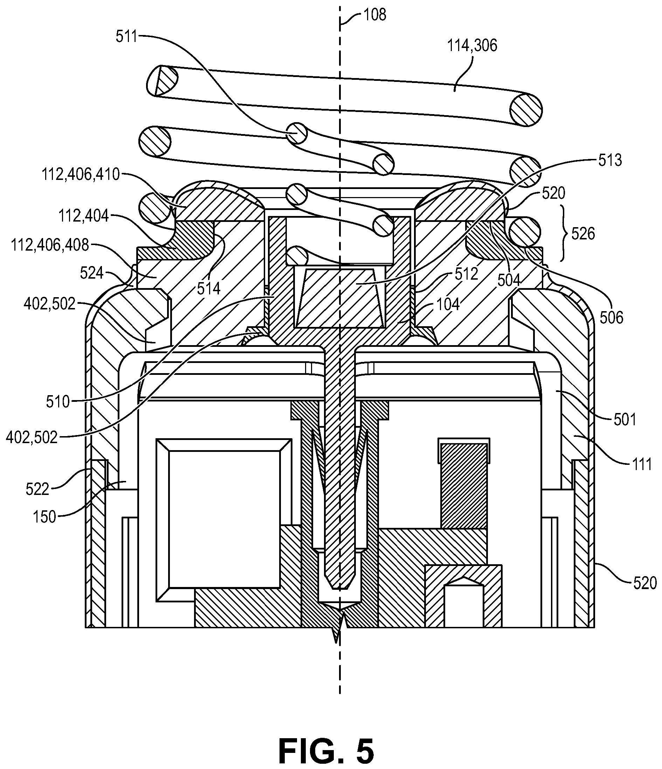

In one implementation, an electronics assembly 152 (shown by hidden lines) is mounted in the electronics compartment 150. The electronics assembly 152 can include, without limitation, a flexible circuit or a printed circuit board having one or more electronic components mounted on a substrate. For example, the electronics assembly 152 can include one or more processors, capacitors, etc., interconnected by electrical traces, vias, or other electrical connectors. In one implementation, the electronics assembly 152 includes an electrical connector to connect to the electrical feedthrough of the header assembly 109. For example, the electrical connector can be a socket connector to receive an electrode pin of the distal electrode 104 (FIG. 5).

The biostimulator components, e.g., the energy source container, the electronics compartment 150 containing the electronics assembly 152, and the header assembly 109, can be arranged on the central axis 108. Accordingly, each component can extend along the central axis 108 and have a respective axial location relative to another component along the central axis 108. For example, the energy source container can be offset from the electronics compartment 150 in a proximal direction 154 and the header assembly 109 can be offset from the electronics compartment 150 in a distal direction 156.

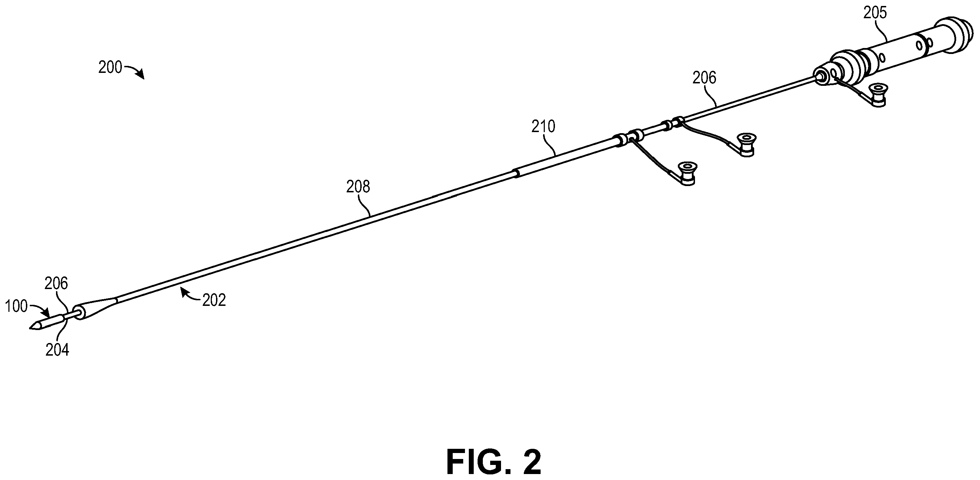

Referring to FIG. 2, a perspective view of a biostimulator system is shown in accordance with an embodiment. Leadless pacemakers or other leadless biostimulators can be delivered to or retrieved from a patient using delivery or retrieval systems. The leadless biostimulator system 200 can include delivery or retrieval systems, which may be catheter-based systems used to carry a leadless biostimulator 100 intravenously to or from a patient anatomy. The delivery or retrieval systems may be referred to collectively as transport systems, or biostimulator transport systems.

A biostimulator system 200 can include a biostimulator transport system 202. The biostimulator 100 can be attached, connected to, or otherwise mounted on the biostimulator transport system 202. For example, the biostimulator transport system 202 can have a distal end 204, and the biostimulator 100 can be mounted on the distal end 204. The biostimulator 100 can thereby be advanced intravenously into or out of the heart.

The biostimulator transport system 202 can include a handle 205 to control movement and operations of the biostimulator transport system 202 from outside of a patient anatomy. One or more elongated members extend distally from the handle 205. For example, a support member 206 can extend distally from the handle 205. The support member 206 can extend to the distal end 204 of the biostimulator transport system 202. In an embodiment, the biostimulator 100 is mounted on the biostimulator transport system 202, e.g., at the distal end 204 of the support member 206.

The biostimulator transport system 202 can include a protective sleeve 208 to cover the biostimulator 100 during delivery and implantation. The protective sleeve 208 can extend over, and be longitudinally movable relative to, the support member 206. The biostimulator transport system 202 may also include an introducer sheath 210 that can extend over, and be longitudinally movable relative to, the protective sleeve 208. The introducer sheath 210 can cover a distal end of the protective sleeve 208, the support member 206, and the biostimulator 100 as those components are passed through an access device into the patient anatomy.

Several components of the biostimulator transport system 202 are described above by way of example. It will be appreciated, however, that the biostimulator transport system 202 may be configured to include additional or alternative components. More particularly, the biostimulator transport system 202 may be configured to deliver and/or retrieve the biostimulator 100 to or from the target anatomy. Delivery and/or retrieval of the biostimulator 100 can include retaining the biostimulator 100 during transport to the target anatomy and rotation of the biostimulator 100 during implantation of the biostimulator 100 at the target anatomy. Accordingly, the biostimulator transport system 202 can incorporate features to retain and rotate the biostimulator 100.

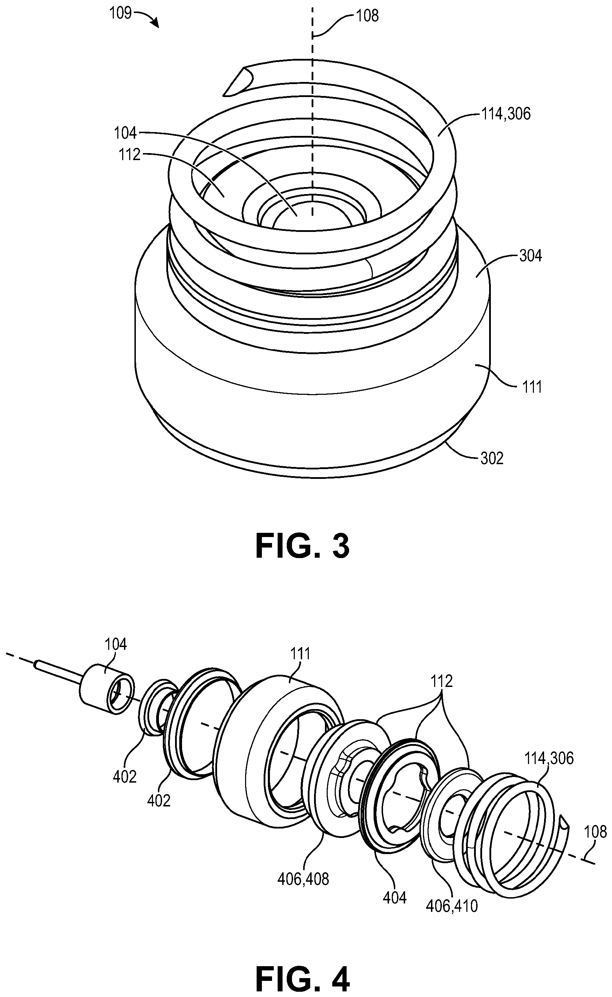

Referring to FIG. 3, a perspective view of a header assembly is shown in accordance with an embodiment. The header assembly 109 can include the flange 111. The flange 111 can have a proximal lip 302, which can mount on the housing wall surrounding the electronics compartment 150. More particularly, a proximal end of the flange 111 can mount on a distal end of the housing 102. In one implementation, the flange 111 is formed from titanium. The flange 111 can be mounted on the housing 102 and connected to the housing 102 by a hermetic seal, e.g., a weld or any other similar hermetically sealed connection. For example, the hermetic weld can be formed circumferentially around a seam between the proximal end of the flange 111, e.g., the proximal lip 302, and the distal end of the housing 102.

In an embodiment, the flange 111 includes a shoulder 304. The shoulder 304 can be a transition region between a flange wall that extends longitudinally from the proximal lip 302 to a flange wall that extends transversely to form a mounting wall. The mounting wall can receive the fixation element mount 112 (FIG. 5) thereon.

In one implementation, the header assembly 109 includes a fixation element 114 mounted on the fixation element mount 112. The fixation element 114 can include a helix 306. The helix 306 can extend distally from the fixation element mount 112 about the central axis 108. For example, the helix 306 can revolve about the central axis 108. The helix 306 can include a spiral wire, formed by coiling or cut from a wall of a length of tubing, which extends in a rotational direction around the central axis 108. For example, the helix 306 can revolve in a right-handed direction about the central axis 108. The helix 306 can be suitable for attaching the biostimulator 100 to tissue, such as heart tissue. For example, in the case of a right-handed spiral direction, the biostimulator 100 can be advanced into contact with a target tissue, and the biostimulator 100 can then be rotated in the right-handed direction to screw the helix 306 into the tissue. Torque can be transmitted from the housing 102 to the helix 306 through the fixation element mount 112, and thus, mechanical stability of the components of the header assembly 109 facilitates torque transmission. In an embodiment, the fixation element mount 112 can be attached to the flange 111 by a brazing joint, as described below, to provide mechanical stability.

Referring to FIG. 4, an exploded view of a header assembly is shown in accordance with an embodiment. In the exploded view, components of the header assembly 109 are seen spaced apart along the central axis 108, e.g., coaxially along the central axis 108. The components include the distal electrode 104, the flange 111, and subcomponents of the fixation element mount 112. The header assembly 109 also includes the fixation element 114, which is optionally the helix 306. The components of the header assembly 109 can be interconnected by one or more bonds. For example, the header assembly 109 can include one or more threadless joints 402, e.g., brazed joints. The bonds may alternatively or additionally include fused ceramic bonding (or pre-formed profile ceramic, alternatively) to integrate the components. For example, the fixation element mount 112 can include a mounting ring 404 on an insulator base 406, and the insulator base 406 may be further divided into a first base ring 408 and a second base ring 410. The base rings 408, 410 can be fused to form the insulator base 406. Such threadless joints 402 can reduce manufacturing process steps and overall device length. The components of the header assembly 109 and their interconnections are described in more detail below.

Referring to FIG. 5, a cross-sectional view of a header assembly is shown in accordance with an embodiment taken along a plane coincident with a central axis of the header assembly. The fixation element mount 112 can be mounted on the flange 111. For example, a portion of the fixation element mount 112, e.g., the insulator base 406, can be placed within a central hole 512 of the flange 111 and joined to the flange 111 by a threadless joint 402. The threadless joint 402 can be a hermetic joint 502, such as a brazed joint formed by a gold braze between the flange 111 and surfaces of the insulator base 406. The hermetic joint 502 can connect the fixation element mount 112 to the flange 111, and can prevent the ingress or egress of fluids between the electronics compartment 150 and a surrounding environment.

The fixation element mount 112, which can be seated on the flange 111 as a means of interconnecting the flange 111 and the housing 102 to the fixation element 114, can include the mounting ring 404 on the insulator base 406. The insulator base 406 can be formed from a ceramic, e.g., alumina, ruby, glass, or another insulating material. Accordingly, the insulator base 406 may be formed from a different material than the fixation element 114, which can be formed from stainless steel, for example. As a result, the insulator base 406 may not be welded to the fixation element 114. By contrast, the mounting ring 404 of the fixation element mount 112 may be compatible with welding to the fixation element 114. For example, the mounting ring 404 and the fixation element 114 may be formed from like materials. Accordingly, whereas the insulator base 406 may be brazed to the flange 111, the mounting ring 404 may be welded to the fixation element 114. The fixation element mount 112 can therefore interconnect the fixation element 114 and the flange 111.

Like the fixation element 114, the mounting ring 404 may also be incompatible with welding to the insulator base 406. The mounting ring 404 may instead be coupled to the insulator base 406 by a threadless, non-thermal connection. For example, the mounting ring 404 can be sandwiched between subcomponents, e.g., the first base ring 408 and the second base ring 410, of the mounting ring 404. In such case, there may be chemical attachment between the base rings 408, 410, and the mounting ring 404 may be joined to the base rings 408, 410 by physical interferences between the nested components.

In an embodiment, several ceramic preform components are fused together to form the insulator base 406. More particularly, the first base ring 408 can be fused to the second base ring 410 by a sintering process. The metal mounting ring 404 can be stacked in between the two green-state ceramic preform components prior to fusing. When stacked, the assembly can be exposed to an elevated temperature environment to promote fused ceramic bonding. The resulting fixation element mount 112 includes the mounting ring 404 extending around the central axis 108 within a groove 504 of the insulator base 406. The groove 504 can be a space between the first and second base rings 408, 410 within which the mounting ring 404 was nested during the fusing process.

The fixation element 114 can be attached to the mounting ring 404. In an embodiment, the fixation element 114 is attached to the mounting ring 404 by a weld. For example, the helix 306 may be directly welded to the mounting ring 404 by a laser weld 506. The direct laser-welding of the fixation element 114 to the fixation element mount 112 may be contrasted with threading the helix 306 onto a groove of a fixation element mount 112. For example, the direct weld 506 can allow the fixation element 114 to have unobstructed spaces longitudinally between helical turns and radially inward of the turns between the helix 306 and the mounting ring 404, e.g., the second base ring 410. Such spaces may allow for secure capture of target tissue by the fixation element 114.

In an embodiment, the electrode distal 104 may include an electrode body 510 and/or an electrode tip 511. In implementations of the present disclosure, the electrode tip 511 may be mounted on the electrode body 510, e.g., on a distal end of the electrode body 510. The distal electrode 104 can be disposed within the central hole 512 of the fixation element mount 112. More particularly, the fixation element mount 112 can include the central hole 512 aligned with the central axis 108, and aligned with a central channel 501 of the housing 102. The distal electrode 104 can be mounted within the central hole 512 along the central axis 108. In an embodiment, the central hole 512 is within the insulator base 406 and, thus, the distal electrode 104 can be electrically isolated from conductive portions of the header assembly 109, such as the flange 111 or the mounting ring 404.

Feedthrough assemblies in accordance with the present disclosure may include a monolithic electrode body 510. For example, the monolithic electrode body 510 can have several distinct portions that are integrally formed with each other. In one implementation, the electrode body 510 includes a cup and a pin that are integrally formed such that the electrode body 510 is monolithic, or, in other words, has a unitary or single-piece construction. More particularly, the cup and the pin can be formed from a single blank of material to produce the electrode body 510 such that the electrode body 510 does not have any seams, welds, etc. The cup can be sized and dimensioned to fit through the central hole 512 in the insulator base 406, and the pin can extend proximally from the cup into the electronics compartment 150 within the housing 102. Accordingly, the monolithic electrode body 510 provides an electrical pathway from the electronics compartment 150, which is proximal to the insulator base 406, to the cup.

The pin and the cup can also serve as the electrically active path from the electronics assembly 152 within the electronics compartment 150 to the patient-contacting pacing electrode tip 511. The integrally formed cup and pin can be of the same material. For example, and without limitation, the electrode body 510 can be formed from 90/10 platinum/iridium alloy or another suitable conductive alloy. The electrode tip 511 may be a helical fixation element 114, as shown. In an embodiment, the helical fixation element 114 is also conductive, e.g., formed from MP35N, and can be physically and/or electrically connected to the cup, e.g., by a thermal weld. During implantation, the electrode tip 511 can contact target tissue, e.g., by screwing into the target tissue. Accordingly, electrical signals delivered to the pin from the circuitry within the electronics compartment 150 can travel through the electrode body 510 and the electrode tip 511 into the target tissue.

The biostimulator 100, and more particularly the header assembly 109, can include a filler 513, such as a monolithic controlled release device (MCRD). The filler 513 may include a therapeutic material, and can be loaded into the cup of the electrode body 510. Accordingly, the filler 513 can deliver a specified dose of a therapeutic agent, e.g., a corticosteroid, into target tissue at an implantation site of the biostimulator 100 within a patient. In at least one implementation, the therapeutic agent can include a corticosteroid, such as dexamethasone sodium phosphate, dexamethasone acetate, etc.

When the biostimulator 100 is implanted at the target site, blood can flow into the electrode cavity through an opening in the electrode tip 511, e.g., through an inner lumen surrounded by the helical fixation element 114. The blood can cause the filler 513 to elute the therapeutic agent. Elution of the filler 513 can be controlled by its own geometry, as well as by a size of the electrode cavity and the geometry of the electrode body 510. Accordingly, the therapeutic agent can flow, or weep, from the MCRD through the opening to the target tissue. When the therapeutic agent is consistently released into the target tissue, the controlled dose can reduce inflammation associated with the device implantation.

Still referring to FIG. 5, an inner edge 514 of the mounting ring 404 can be seated in the groove 504. The inner edge 514 may, for example, conform to a surface of the insulator base 406 that faces or extends around the groove 504. Similarly, a portion of the surface of the insulator base 406 surrounding the groove 504, e.g., the upper and lower surfaces facing each other across the groove 504, can appose longitudinally-facing surfaces of the mounting ring 404 (upper and lower disc faces). Accordingly, the mounting ring 404 can be captured in the groove 504. A portion of the mounting ring 404, however, can be exposed radially outward from the insulator base 406 and the groove 504. For example, as described above, the portion of the mounting ring 404 to which the fixation element 114 is directly welded may be exposed from the groove 504 to receive the fixation element 114.

The mounting ring 404 may be captured in the groove 504 between subcomponents of the fixation element mount 112. For example, the mounting ring 404 may be longitudinally between the first base ring 408 and the second base ring 410. The base ring portions 408, 410 can sandwich the mounting ring 404.

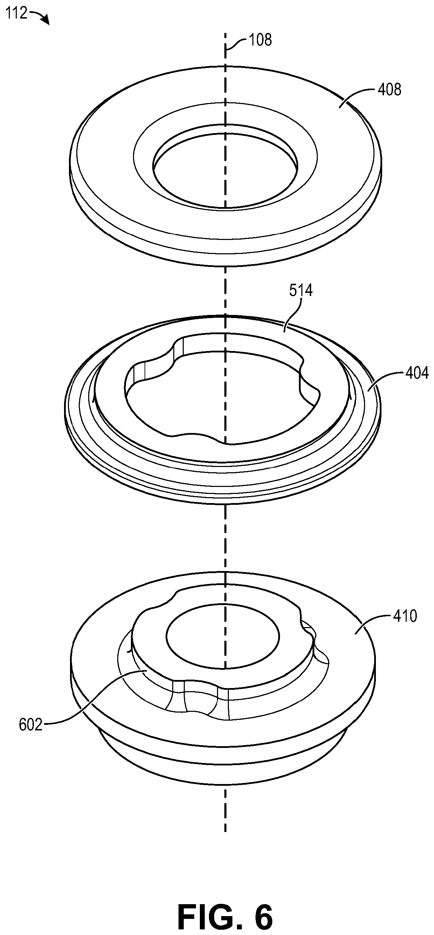

Referring to FIG. 6, an exploded view of a fixation element mount is shown in accordance with an embodiment. In an embodiment, the first base ring 408 and the second base ring 410 are green-state ceramic preform components prior to a fusing process. For example, the subcomponents can be alumina preforms, e.g., preforms shaped from 99% pure Al₂O₃, that can bond to each other when placed in contact and fused. More particularly, the base rings 408, 410 can be fused together while sandwiching the mounting ring 404 to form the fixation element mount 112. The mounting ring 404 may therefore incorporate the insulator base 406 having the first base ring 408 fused to the second base ring 410 to sandwich the mounting ring 404 between the preforms. The fused preforms can cradle and retain the mounting ring 404 such that the mounting ring 404 is longitudinally constrained between the first base ring 408 and the second base ring 410.

The mounting ring 404 can include the inner edge 514 that is captured within the groove 504. The inner edge 514 may be sized and dimensioned to fit around and slip over an outer edge 602 of the second base ring 410, for example. Accordingly, lateral movement of the mounting ring 404 may be constrained by interference between the concentrically located inner edge 514 and outer edge 602.

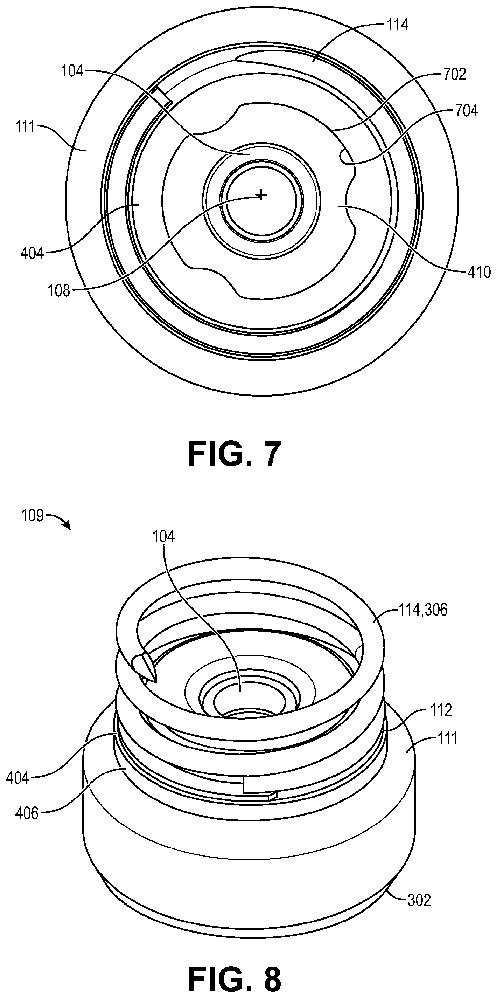

Referring to FIG. 7, an end view of a header assembly is shown in accordance with an embodiment. In addition to constraining longitudinal movement of the mounting ring 404, the insulator base 406 can constrain rotational movement of the mounting ring 404. In an embodiment, the groove 504 includes an outer groove profile 702, e.g., a cross-sectional profile of the outer edge 602. Similarly, the mounting ring 404 includes an inner ring profile 704, e.g., a cross-sectional profile of the inner edge 514. The profiles can be shaped to restrict relative movement between the respective components. More particularly, the outer groove profile 702 and the inner ring profile 704 can be shaped to interfere with rotation of the mounting ring 404 about the central axis 108 relative to the insulator base 406.

The anti-rotation feature of the fixation element mount 112 can include a keyed connection between the insulator base 406 and the fixation element mount 112. For example, one of the components can include a key that fits into a keyway of the other component. The key can engage the keyway such that rotation between the components is synchronized and, more particularly, one component does not rotate relative to the other.

In an embodiment, the components include several mating keys and keyways around the central axis 108. For example, the fixation element mount 112 can have several, e.g., three lobes, evenly distributed in a circumferential direction about the central axis 108. More particularly, the lobes may be separated from each other by valleys located at 120 degree increments around the central axis 108. Corresponding lobes of the fixation element mount 112 can extend into the valleys, creating the interference to resist movement between the components.

Referring again to FIG. 5, the components of the header assembly 109 may be hermetically joined to prevent fluid from leaking between the electronics compartment 150 and a surrounding environment. In an embodiment, the electrode body 510, the insulator base 406, and the flange 111 are joined by a brazing process with melted gold preforms occupying the interface between the components. More particularly, as described above, the threadless joints 402 can be brazed in between the gaps that separate the components. The device hermeticity maintained by the gold braze joints 402 between the components can reduce the likelihood of electrical leaking as well. Electricity can leak from the circuitry within the electronics compartment 150 into the surrounding environment through fluid paths. Sealing off (eliminating) such fluid paths can reduce or eliminate electrical leaks. The hermetically sealed header assembly 109 can therefore limit electrical passage through the electrode body 510, which can provide consistent and sustainable device functionality.

Fluid and electrical leaks are reduced and/or avoided because the threaded coupling between components of the header assembly 109 are eliminated. More particularly, leaks can occur between threads and, thus, eliminating threads can eliminate leaks. Eliminating threads may also contribute to a minimized device length, e.g., along the central axis 108, or width, e.g., diameter. More particularly, given that threads require substantial surface contact in a longitudinal direction, eliminating the threads can reduce the length of the header assembly 109. By way of example, it has been shown that replacing the thread-locking mechanism of existing designs with the brazing and weld joints described above, can reduce an overall length of the header assembly 109 by 25%, e.g., from 0.182 (0,46 cm) inch to 0.142 (0,36 cm) inch. Advantageously, the reduced length of the header assembly 109 may allow the biostimulator 100 to fit within smaller anatomies.

The header assembly 109 may improve electrical isolation both by eliminating threaded connections and by eliminating seal components, such as gaskets. The fewer number of seal components can reduce potential leak paths. The reduced potential for leak paths also reduces the potential of electrical leakage.

Electrical isolation of the header assembly 109 may also be promoted using an electrically insulating coating. In an embodiment, an insulative coating 520 can cover an outer flange surface 522 of the flange 111. The insulative coating 520 may also cover an outer base surface 524 of the insulator base 406. The outer surfaces of the flange 111 and the insulator base 406 can include the surfaces that are facing outward toward the surrounding environment. More particularly, the outward surfaces facing away from the central channel 501 and the central hole 512 can be coated by the insulative coating 520. The insulative coating 520 may be parylene or another dielectric material, for example. The insulative coating 520 can cover components at the outer surfaces, e.g., between the second base ring 410 and the flange 111, for example. Accordingly, a risk of fluid and/or electrical leakage can be reduced.

In an embodiment, the insulative coating 520 has a break in the film over the mounting ring 404. More particularly, the mounting ring 404 may be exposed through a gap 526 in the insulative coating 520. The gap 526 in the insulative coating 520 can allow for the weld 506 between the fixation element 114 and the fixation element mount 112 to be made. More particularly, an external surface of the fixation element mount 112 may remain uncoated to allow for contact between the exposed surfaces of the fixation element 114 and the fixation element mount 112. The fixation element mount 112, however, can be cradled and insulated by the insulator base 406. Accordingly, metal of the fixation element mount 112 and the fixation element 114 does not create an electrical leak risk.

Referring to FIG. 8, a perspective view of a header assembly is shown in accordance with an embodiment. As described above, the header assembly 109 may have an annular mounting ring 404. Alternatively, the ring may be a partial ring, e.g., C-shaped. Such an embodiment is described below. The header assembly 109 of FIG. 8 can have components similar or the same as those illustrated in FIG. 3. For example, the header assembly 109 can include the flange 111. The flange 111, which can mount on the housing 102 wall surrounding the electronics compartment 150. The flange 111 can receive the fixation element mount 112. More particularly, the insulator base 406 may be mounted on the flange 111, and the mounting ring 404 may be mounted on the insulator base 406. In one implementation, the fixation element 114, which may be attached, e.g., welded, to the mounting ring 404 can include the helix 306. The distal electrode 104 may be concentrically arranged with the insulator base 406 and the mounting ring 404, e.g., within the central hole 512 of the insulator base 406. The components of the header assembly 109 may be physically connected and hermetically sealed by the threadless joints 402, e.g., brazing joints, as described above.

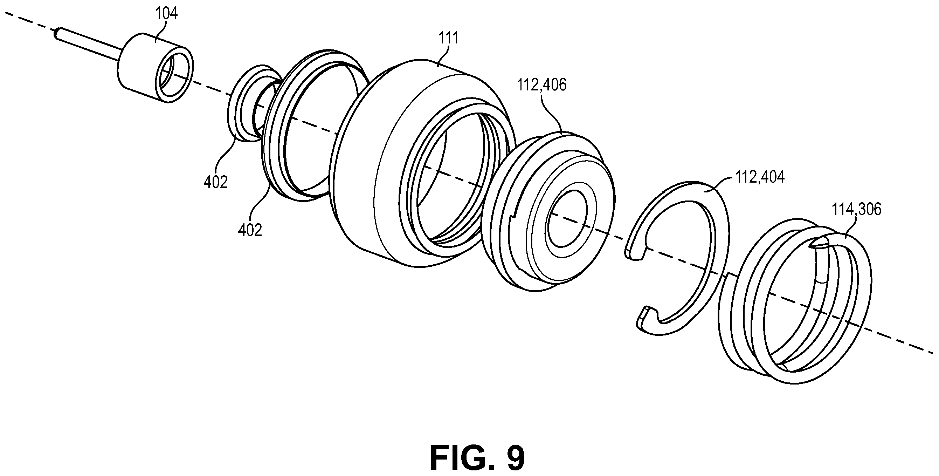

Referring to FIG. 9, an exploded view of a header assembly is shown in accordance with an embodiment. The header assembly 109 may be configured similar to the embodiment illustrated in FIG. 5. For example, the insulator base 406 can be mounted on and sealed to the flange 111 by brazing the threadless joint 402. Similarly, the distal electrode 104 may be physically attached to the insulator base 406 by brazing the threadless joint 402. The threadless joints 402 can form a hermetic seal between the components to isolate the electronics compartment 150, while allowing electrical signals to be communicated from the circuitry within the electronics compartment 150 through the electrode body 510 and tip to the surrounding environment (and the target tissue). It will be appreciated that, by comparison to FIG. 5, similar components can have similar structure and function, and the description is not repeated here in the interest of brevity.

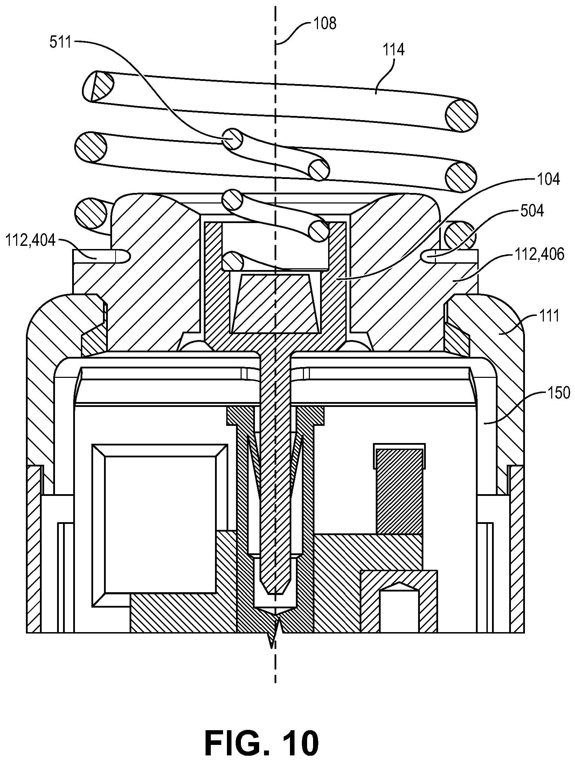

Referring to FIG. 10, a cross-sectional view of a header assembly is shown in accordance with an embodiment taken along a plane coincident with a central axis of the header assembly. Key differences between the embodiments of FIGS. 5 and 10 can include the structures of the insulator base 406 and the mounting ring 404. In an embodiment, the insulator base 406 is a singular ceramic insulator component that can be pre-formed in a green state prior to sintering. The preform of the insulator base 406 can include an undercut, e.g., the groove 504, to receive the mounting ring 404. More particularly, rather than being formed between subcomponents of the insulator base 406 when the subcomponents are fused together, the groove 504 can be pre-formed in the insulator base 406 or introduced in a secondary cutting operation. As a result, the mounting ring 404 may be installed in the groove 504 after the insulator base 406 is sintered, rather than before such as in the case of the embodiment shown in FIG. 5.

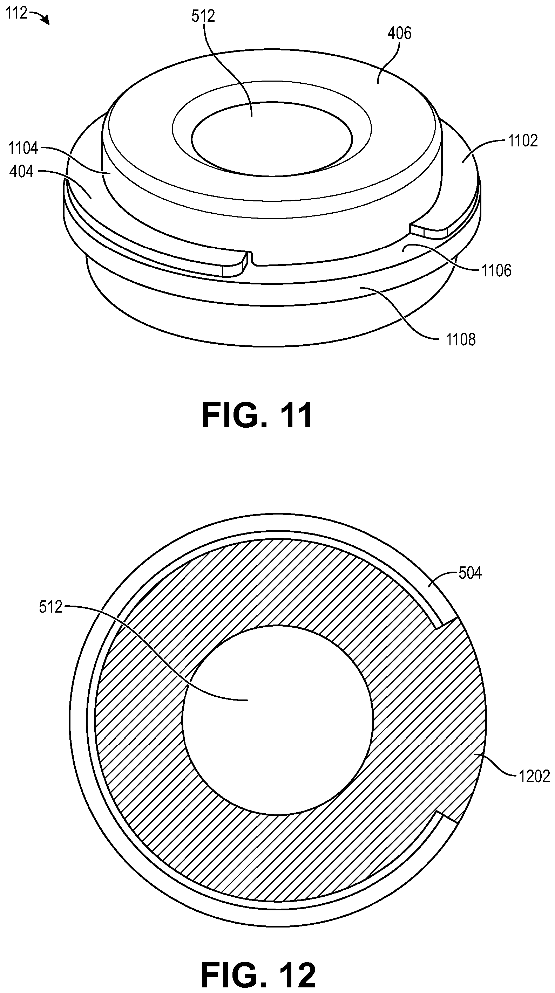

Referring to FIG. 11, a perspective view of a fixation element mount is shown in accordance with an embodiment. The mounting ring 404 may be a flexible metal insert, or clip, having an opening. For example, the mounting ring 404 may be a partial ring have a C-shaped profile. The ends of the C-shape can be spaced apart in a circumferential direction such that, when the mounting ring 404 is deformed, the ends can move apart from each other. Accordingly, the C-shaped mounting ring 404 can be clipped onto the insulator base 406.

When the flexible mounting ring 404 is clipped onto (or into) the undercut feature of the insulator base 406, it can provide a mounting surface 1102 onto which the fixation element 114 can be bonded. For example, the helix 306 can be installed over the insulator base 406, e.g., around the cylindrical distal hub 1104 of the insulator base 406, and welded to the mounting ring 404.

The header assembly 109 can include a bond between the mounting ring 404 and the insulator base 406. In an embodiment, a titanium nickel (TiNi) preform brazing ring 1106 can be installed on a ledge 1108 of the insulator base 406. The ledge 1108 can be a circular shelf on which the mounting ring 404 rests when it is clipped into the undercut. The brazing ring 1106 can be used to join the metal mounting ring 404 to the ceramic insulator base 406. For example, after clipping the flexible mounting ring 404 onto the insulator base 406, the assembly can be heated to fuse the mounting ring 404 to the insulator base 406 by the melted brazing ring 1106.

Referring to FIG. 12, a cross-sectional view of an insulator base is shown in accordance with an embodiment taken along a plane defined by a ledge of the insulator base. The insulator base 406 and/or mounting ring 404 can include an anti-rotation feature. The insulator base 406 can include the groove 504 formed in a sidewall of the distal hub 1104. The groove 504 can extend around less than a full circumference of the hub, e.g., over a 300 degree arc. More particularly, the groove 504 can be semi-circular groove having an arc length equal to an arc length of the mounting ring 404. The semi-circular groove 504 can therefore have groove ends that are separated by a key portion 1202. For example, the key portion 1202 can be a 60 degree length of material that fits between the ends of the C-shaped mounting ring 404. The key portion 1202 can interfere with the ends of the mounting ring 404 and resist rotational movement of the mounting ring 404 relative to the insulator base 406 when the mounting ring 404 is installed in the groove 504.

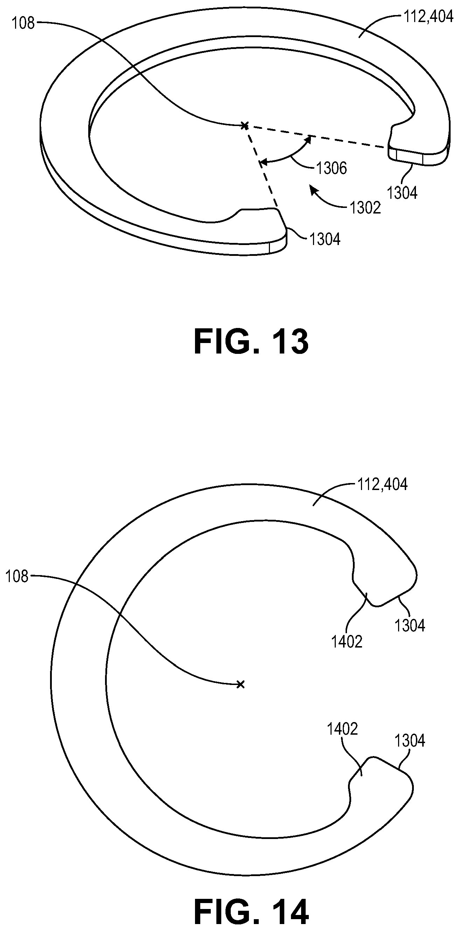

Referring to FIG. 13, a perspective view of a mounting ring is shown in accordance with an embodiment. The mounting ring 404 can include the anti-rotation feature. As described above, the mounting ring 404 can have a partial annular shape. More particularly, the mounting ring 404 can extend along a circular profile, however, an opening 1302 can exist between ring ends 1304. The opening 1302 may have a size that allows it to engage and slide over the insulator base 406 into the groove 504. For example, an arc angle 1306 of the opening 1302 may be in a range of 45 to 75 degrees, e.g., 60 degrees.

Referring to FIG. 14, a top view of a mounting ring is shown in accordance with an embodiment. The opening 1302 creates a C-shaped profile, as shown. In an embodiment, the mounting ring 404 includes one or more teeth extending radially inward toward the central axis 108. For example, the mounting ring 404 can include a tooth 1402 at the ring end 1304. In an embodiment, the mounting ring 404 includes teeth 1402 at each of the ring ends 1304. The teeth 1402 can extend toward the central axis 108 into mating recesses (not shown) formed in the insulator base 406. When the mounting ring 404 is clipped over the insulator base 406, the C-shaped profile can flex outward until the distal hub 1104 is captured within an interior of the mounting ring 404. The teeth 1402 can then snap into the recesses in the insulator base 406. Rotational movement of the mounting ring 404 relative to the insulator base 406 can be impeded by interference between the teeth 1402 and surfaces surrounding the recess of the insulator base 406.

The header assembly 109 described above can simplify manufacturing relative to existing header assemblies. Eliminating gaskets and seals and introducing bonding processes that may be automated can eliminate manufacturing steps, save time, and reduce manufacturing costs. For example, there may be no need to manually insert gaskets into the header assembly 109, load or align the helix 306 onto a threaded mount, or screw such mount onto the flange 111. Similarly, steps to align the helix 306 on the threaded mount can be eliminated. The resulting simplified process can produce the header assembly 109 having the integrated fixation element mount 112 using fewer components and less time.

In an embodiment, a manufacturing process includes forming the fixation element mount 112, including fusing the insulator base 406 with the mounting ring 404 in the groove 504 and/or loading the mounting ring 404 over the insulator base 406 into the groove 504. The insulator base 406 may be attached to the flange 111 by a brazing joint 402 to connect the fixation element mount 112 to the flange 111. Similarly, the distal electrode 104 may be loaded into the central hole 512 of the insulator base 406 and joined to the fixation element mount 112 by a brazing joint 402. The flange 111 can be connected to the housing 102, e.g., by a weld. The housing 102 can contain circuitry of the electronics assembly 152. A coating, e.g., parylene, can be applied over the housing 102 and portions of the header assembly 109, as described above. The filler 513 can be loaded into the distal electrode 104. After the header assembly 109 is attached to the housing 102, the fixation element 114, e.g., the helix 306, can be loaded onto the mounting ring 404 and fixed by the weld 506. The manufacturing process described above provides a simple, low-cost process to produce a header assembly 109 having miniaturized length, and hermetically sealed and electrically isolated components.

In the foregoing specification, the invention has been described with reference to specific exemplary embodiments thereof. It will be evident that various modifications may be made thereto without departing from the scope of the invention as set forth in the following claims. The specification and drawings are, accordingly, to be regarded in an illustrative sense rather than a restrictive sense.

## Claims

1. A header assembly (109) for a biostimulator (100), comprising:
a flange (111) having a central axis (108);
a fixation element mount (112) mounted on the flange (111), wherein the fixation element mount (112) includes a mounting ring (404) on an insulator base (406), and wherein the mounting ring (404) extends around the central axis (108) within a groove (504) of the insulator base (406); and
a fixation element (114) coupled to the mounting ring (404).

2. The header assembly of claim 1, wherein the fixation element mount (112) is coupled to the flange (111) by a hermetic joint (502).

3. The header assembly of claim 1 or 2, wherein the fixation element mount (112) includes a central hole (512) aligned with the central axis (108), and further comprising an electrode (104) mounted within the central hole (512) along the central axis (108).

4. The header assembly of any one of claims 1 to 3, wherein the fixation element (114) includes a helix (306) revolving about the central axis (108).

5. The header assembly of any one of claims 1 to 4, wherein the fixation element (114) is coupled to the mounting ring (404) by a weld (506).

6. The header assembly of any one of claims 1 to 5, wherein an inner edge (514) of the mounting ring (404) is seated in the groove (504).

7. The header assembly of any one of claims 1 to 6, wherein the insulator base (406) includes a first base ring (408) fused to a second base ring (410).

8. The header assembly of claim 7, wherein the mounting ring (404) is between the first base ring (408) and the second base ring (410).

9. The header assembly of any one of claims 1 to 8, wherein the mounting ring (404) has a C-shaped profile.

10. The header assembly of claim 9, wherein the C-shaped profile includes a tooth (1402) extending radially inward toward the central axis (108) at a ring end (1304).

11. The header assembly of any one of claims 1 to 10, wherein an outer groove profile (702) of the groove (504) and an inner ring profile (704) of the mounting ring (404) are shaped to interfere with rotation of the mounting ring (404) about the central axis (108) relative to the insulator base (406).

12. The header assembly of any one of claims 1 to 11, further comprising an insulative coating (520) covering an outer flange surface (522) of the flange (111) and an outer base surface (524) of the insulator base (406).

13. The header assembly of claim 12, wherein the mounting ring (404) is exposed through a gap (526) in the insulative coating (520).

14. A biostimulator (100), comprising:
a housing (102) having a central axis (108) and an electronics compartment (150);
an electronics assembly (152) mounted in the electronics compartment (150); and
the header assembly (109) of any of claims 1 to 13, wherein the flange (111) is mounted on the housing (102) along the central axis (108).

15. A biostimulator system (200), comprising:
a biostimulator transport system (202) having a distal end (204); and
the biostimulator (100) of claim 14 coupled to the distal end (204) of the biostimulator transport system (202).

## Patentansprüche

1. Kopfbaugruppe (109) für einen Biostimulator (100), umfassend:
einen Flansch (111), der eine zentrale Achse (108) aufweist;
eine Fixierungselementhalterung (112), die an dem Flansch (111) montiert ist, wobei die Fixierungselementhalterung (112) einen Halterungsring (404) auf einer Isolatorbasis (406) beinhaltet und wobei sich der Halterungsring (404) innerhalb einer Nut (504) der Isolatorbasis (406) um die zentrale Achse (108) erstreckt; und
ein Fixierungselement (114), das an den Halterungsring (404) gekoppelt ist.

2. Kopfbaugruppe nach Anspruch 1, wobei die Fixierungselementhalterung (112) durch eine hermetische Verbindung (502) an den Flansch (111) gekoppelt ist.

3. Kopfbaugruppe nach Anspruch 1 oder 2, wobei die Fixierungselementhalterung (112) ein zentrales Loch (512) beinhaltet, das mit der zentralen Achse (108) ausgerichtet ist, und ferner umfassend eine Elektrode (104), die innerhalb des zentralen Lochs (512) entlang der zentralen Achse (108) montiert ist.

4. Kopfbaugruppe nach einem der Ansprüche 1 bis 3, wobei das Fixierungselement (114) eine Schrägung (306) beinhaltet, die sich um die zentrale Achse (108) dreht.

5. Kopfbaugruppe nach einem der Ansprüche 1 bis 4, wobei das Fixierungselement (114) durch eine Schweißung (506) an den Halterungsring (404) gekoppelt ist.

6. Kopfbaugruppe nach einem der Ansprüche 1 bis 5, wobei eine innere Kante (514) des Halterungsrings (404) in der Nut (504) sitzt.

7. Kopfbaugruppe nach einem der Ansprüche 1 bis 6, wobei die Isolatorbasis (406) einen ersten Basisring (408) beinhaltet, der mit einem zweiten Basisring (410) fusioniert ist.

8. Kopfbaugruppe nach Anspruch 7, wobei sich der Halterungsring (404) zwischen dem ersten Basisring (408) und dem zweiten Basisring (410) befindet.

9. Kopfbaugruppe nach einem der Ansprüche 1 bis 8, wobei der Halterungsring (404) ein C-förmiges Profil aufweist.

10. Kopfbaugruppe nach Anspruch 9, wobei das C-förmige Profil einen Zahn (1402) beinhaltet, der sich an einem Ringende (1304) radial nach innen zu der zentralen Achse (108) erstreckt.

11. Kopfbaugruppe nach einem der Ansprüche 1 bis 10, wobei ein äußeres Nutprofil (702) der Nut (504) und ein inneres Ringprofil (704) des Halterungsrings (404) dazu geformt sind, eine Drehung des Halterungsrings (404) um die zentrale Achse (108) relativ zu der Isolatorbasis (406) zu stören.

12. Kopfbaugruppe nach einem der Ansprüche 1 bis 11, ferner umfassend eine isolierende Beschichtung (520), die eine äußere Flanschoberfläche (522) des Flansches (111) und eine äußere Basisoberfläche (524) der Isolatorbasis (406) bedeckt.

13. Kopfbaugruppe nach Anspruch 12, wobei der Halterungsring (404) durch einen Spalt (526) in der isolierenden Beschichtung (520) freigelegt ist.

14. Biostimulator (100), umfassend:
ein Gehäuse (102), das eine zentrale Achse (108) und ein Elektronikfach (150) aufweist;
eine Elektronikbaugruppe (152), die in dem Elektronikfach (150) montiert ist; und
die Kopfbaugruppe (109) nach einem der Ansprüche 1 bis 13, wobei der Flansch (111) entlang der zentralen Achse (108) an dem Gehäuse (102) montiert ist.

15. Biostimulatorsystem (200), umfassend:
ein Biostimulatortransportsystem (202), das ein distales Ende (204) aufweist; und
den Biostimulator (100) nach Anspruch 14, der an das distale Ende (204) des Biostimulatortransportsystems (202) gekoppelt ist.

## Revendications

1. Ensemble collecteur (109) pour un biostimulateur (100), comprenant :
une bride (111) présentant un axe central (108) ;
un support d'élément de fixation (112) monté sur la bride (111), dans lequel le support d'élément de fixation (112) comprend un anneau de montage (404) sur une base isolante (406), et dans lequel l'anneau de montage (404) s'étend autour de l'axe central (108) à l'intérieur d'une rainure (504) de la base isolante (406) ; et
un élément de fixation (114) relié à l'anneau de montage (404).

2. Ensemble collecteur selon la revendication 1, dans lequel le support d'élément de fixation (112) est relié à la bride (111) par un joint hermétique (502).

3. Ensemble collecteur selon la revendication 1 ou 2, dans lequel le support d'élément de fixation (112) comprend un orifice central (512) aligné avec l'axe central (108), et comprenant en outre une électrode (104) montée à l'intérieur de l'orifice central (512) le long de l'axe central (108).

4. Ensemble collecteur selon l'une quelconque des revendications 1 à 3, dans lequel l'élément de fixation (114) comprend une hélice (306) s'enroulant autour de l'axe central (108).

5. Ensemble collecteur selon l'une quelconque des revendications 1 à 4, dans lequel l'élément de fixation (114) est relié à l'anneau de montage (404) par une soudure (506).

6. Ensemble collecteur selon l'une quelconque des revendications 1 à 5, dans lequel un bord interne (514) de l'anneau de montage (404) est logé dans la rainure (504).

7. Ensemble collecteur selon l'une quelconque des revendications 1 à 6, dans lequel la base isolante (406) comprend un premier anneau de base (408) uni par fusion à un second anneau de base (410).

8. Ensemble collecteur selon la revendication 7, dans lequel l'anneau de montage (404) est disposé entre le premier anneau de base (408) et le second anneau de base (410).

9. Ensemble collecteur selon l'une quelconque des revendications 1 à 8, dans lequel l'anneau de montage (404) présente un profil en forme de C.

10. Ensemble collecteur selon la revendication 9, dans lequel le profil en forme de C comprend une dent (1402) s'étendant radialement vers l'intérieur en direction de l'axe central (108) au niveau d'une extrémité de l'anneau (1304).

11. Ensemble collecteur selon l'une quelconque des revendications 1 à 10, dans lequel un profil de rainure externe (702) de la rainure (504) et un profil d'anneau interne (704) de l'anneau de montage (404) sont conformés pour entraver une rotation de l'anneau de montage (404) autour de l'axe central (108) par rapport à la base isolante (406).

12. Ensemble collecteur selon l'une quelconque des revendications 1 à 11, comprenant en outre un revêtement isolant (520) recouvrant une surface externe (522) de la bride (111) et une surface externe (524) de la base isolante (406).

13. Ensemble collecteur selon la revendication 12, dans lequel l'anneau de montage (404) est exposé à travers un interstice (526) dans le revêtement isolant (520).

14. Biostimulateur (100), comprenant :
un boîtier (102) présentant un axe central (108) et un compartiment électronique (150) ;
un ensemble électronique (152) monté dans le compartiment électronique (150) ; et
l'ensemble collecteur (109) selon l'une quelconque des revendications 1 à 13, dans lequel la bride (111) est montée sur le boîtier (102) le long de l'axe central (108).

15. Système de biostimulateur (200), comprenant :
un système de transport de biostimulateur (202) présentant une extrémité distale (204) ; et
le biostimulateur (100) selon la revendication 14 relié à l'extrémité distale (204) du système de transport de biostimulateur (202).
